# EUROPEAN PATENT SPECIFICATION

(11) **EP 2 895 228 B1**
(45) Date of publication and mention of the grant of the patent: **20.11.2019**
(21) Application number: 13770568.7
(22) Date of filing: 11.09.2013
(51) Int. Cl.: A61M 25/00, A61M 25/01, A61M 25/06, A61M 25/10

(54) **LOADING TOOLS FOR USE WITH BALLOON CATHETERS**
LADEWERKZEUGE ZUR VERWENDUNG MIT BALLONKATHETERN
OUTILS DE CHARGEMENT DESTINÉS À ÊTRE UTILISÉS AVEC DES CATHÉTERS À BALLONNET

(30) Priority: 11.09.2012 US 201261699648 P
(43) Date of publication of application: 22.07.2015
(73) Proprietor: Boston Scientific Scimed, Inc., Maple Grove, MN 55311 (US)
(72) Inventor: GUNDERSON, Richard C., St. Paul , MN 55116 (US); GROVENDER, Adam D., Maple Grove, MN 55311 (US); GROVER, Joel P., St. Paul, Minnesota 55117 (US); GULER, Ismail, Maple Grove, MN 55311 (US); SHUROS, Allan C., St. Paul, Minnesota 55116 (US); WANG, Huisun, Maple Grove, Minnesota 55311 (US)
(74) Representative: Peterreins Schley
(86) International application number: PCT/US2013/059278
(87) International publication number: WO 2014/043245

(56) References cited:
- WO-A1-01/02045
- WO-A2-2007/132444
- US-A1- 2002 062 129
- US-A1- 2011 208 284

## Description

### Technical Field

The present disclosure pertains to medical devices, and methods for manufacturing medical devices. More particularly, the present disclosure pertains to loading tools for use with balloon catheters. The present invention provides a loading tool for use with a medical device and a medical device assembly.

### Background

A wide variety of intracorporeal medical devices have been developed for medical use, for example, intravascular use. Some of these devices include guidewires, catheters, and the like. These devices are manufactured by any one of a variety of different manufacturing methods and may be used according to any one of a variety of methods. Of the known medical devices and methods, each has certain advantages and disadvantages. There is an ongoing need to provide alternative medical devices as well as alternative methods for manufacturing and using medical devices.

US 2011/208284 A1 discloses a protective sleeve for a medical device including a main body which has a distal end and a proximal end and on the lateral surface of which at least one stop element is disposed, which during insertion of the protective sleeve into an introducer limits the insertion depth of the protective sleeve into the introducer and which preferably is disposed at the distal end of the main body.

US 2002/062129 A1 discloses a stent delivery catheter comprising an outer sheath, a peel-away sheath, and an inner sheath; and a stent delivery catheter comprising an outer sheath and an inner tubular member which distally ends in a tongue having an arcuate cross section.

WO 01/02045 A1 discloses a protective sleeve for a catheter having a distal end capable of being disposed within and engaging a majority of existing hemostatic valves; and capable of being partially inserted into a patient, thus, securing the sleeve to the patient in a sheathless catheter insertion procedure.

WO 2007/132444 A2 discloses a catheter insertion system utilizing a catheter introducer device having an introducer sheath.

### Brief Summary

The present invention relates to a loading tool for use with a medical device, the loading tool comprising a tubular member configured to be disposed about a balloon catheter, the tubular member including a distal portion, a proximal portion, and a necked down region disposed between the distal portion and the proximal portion; wherein the distal portion has a distal inner diameter and the necked down region includes a necked inner diameter smaller than the distal inner diameter; and characterized in that the proximal portion has a plurality of axial slits formed therein, wherein the axial slits are in the form of slots.

The present invention further relates to a medical device assembly, comprising a balloon catheter including a catheter shaft and a balloon coupled to the catheter shaft; and a loading tool slidably disposed on the catheter shaft, the loading tool including: a tubular member including a distal portion, a proximal portion, and a necked down region disposed between the distal portion and the proximal portion, wherein the distal portion has a distal inner diameter and the necked down region includes a necked inner diameter smaller than the distal inner diameter, and characterized in the proximal portion has a plurality of axial slits formed therein, wherein the axial slits are in the form of slots.

### Brief Description of the Drawings

The disclosure may be more completely understood in consideration of the following detailed description in connection with the accompanying drawings, in which:
Figure 1 is a cross-sectional side view of an example loading tool;
Figure 2 is a side view of an example loading tool in a first configuration;
Figure 3 is a side view of the example loading tool shown in Figure 2 in a second configuration;
Figure 4 is a side view of an example loading tool disposed along a catheter shaft;
Figure 5 is a side view of a portion of an example loading tool disposed about a balloon;
Figure 6 is a side view of use of an example loading tool for advancing a catheter into a medical valve;
Figure 7 is a side view depicting an example loading tool shifted into a second configuration;
Figure 8 is a side view of another example loading tool;
Figure 9 is an end view of a portion of an example loading tool;
Figure 10 is a partial cross-sectional side view of an example loading tool;
Figure 11 is a side view of a portion of an example loading tool disposed about a balloon; and
Figures 12-14 illustrate the use of an example loading tool and the splitting open of the example loading tool.

While the disclosure is amenable to various modifications and alternative forms, specifics thereof have been shown by way of example in the drawings and will be described in detail. It should be understood, however, that the intention is not to limit the invention to the particular embodiments described.

### Detailed Description

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the invention.

Percutaneous angioplasty and the use of balloon catheters are common practices throughout the world. When using balloon catheters, a clinician may come into contact with the balloon. For example, when backloading a guidewire into the balloon catheter and/or when advancing the balloon catheter into a hemostatis valve, introducer, or the like the clinician may grasp or otherwise handle the balloon. For a number of reasons, it may be desirable to minimize contact with the balloon. For example, if the balloon includes a pharmacological coating or stent with a pharmacological coating, handling the balloon could impact the coating. Disclosed herein are a number of devices that help reduce the amount of contact between a clinician and a balloon on a balloon catheter. Also disclosed are assemblies that help reduce contact with the balloon and methods for using (e.g., loading) balloons and/or balloon catheters.

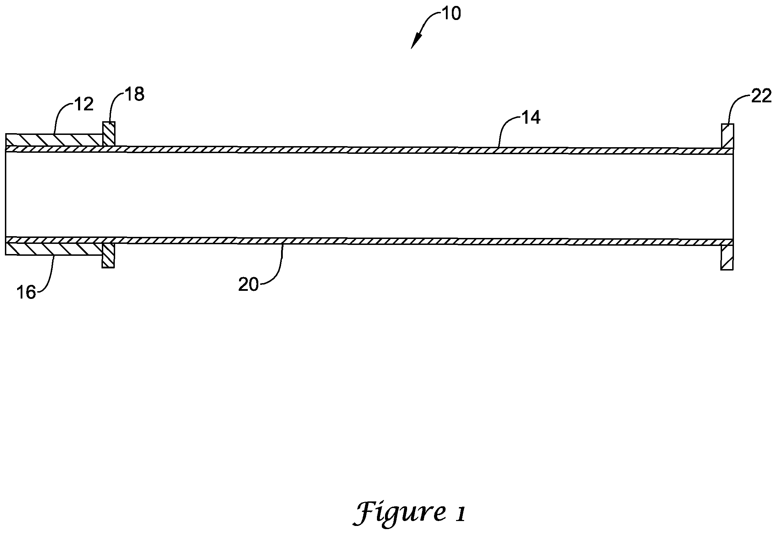

Figure 1 is a side view of an example loading tool 10. Loading tool 10 may generally take the form of a tubular member that includes a distal portion 12 and a proximal portion 14. Distal portion 12 may include a tube body or tube wall 16 and a flange or ridge 18. Proximal portion 14 may also include a tube body or tube wall 20 and a flange or ridge 22. Flanges 18/22 may generally function as stops that prevent loading tool 10 from unintentionally passing too far into other devices such as introducers/dilators/valves and may also serve as handles that aid in the use of loading tool 10. In general, loading tool 10 may be configured to be used with a medical device such as a balloon catheter. For example, a portion of loading tool 10 (e.g., distal portion 12) may disposed over a balloon on a balloon catheter. This may allow a clinician to "handle" the balloon by using loading tool 10 without directly contacting the balloon. Accordingly, other portions of a medical intervention such as guidewire loading/backloading, advancing the balloon catheter through a hemostasis valve, introducer, and/or the like, or other processes may be completed while minimizing contact with the balloon.

In at least some embodiments, tube body 16 may be thicker or otherwise have a larger wall thickness than tube body 20. For example, proximal portion 14 may include a relative thin walled tube body 20 (e.g., about 0.127 mm (0.005 inches) or less, or about 0.102 mm (0.004 inches) or less, or about 0.076 mm (0.003 inches) or less, or about 0.051 mm (0.002 inches) or less, or about 0.0254 mm (0.001 inches) or less, or about 0.0254 mm (0.001 inches)). Tube bodies 16/20 may be formed from a single layer of material. In other embodiments, one or both of tube bodies 16/20 may include a plurality of layers. The layers may include the same or different materials. Some example materials for tube bodies 16/18 (and/or other portions of loading tool 10) may include polyether block amide, nylon, polytetrafluoroethylene, or the like. These are just examples. Other materials are contemplated including those disclosed herein.

Tube body 16 may be formed from a relatively stiffer material than tube body 20. According to one or more of these embodiments, distal portion 12 may be configured to provide structural support when advancing loading tool 10 into a medical valve, introducer, dilator, or the like. For example, when advancing loading tool 10 into a hemostasis valve, the valve may exert some radially inward pressure (e.g., compression) onto loading tool 10. The structural support provided by distal portion 12 may reduce the amount of pressure/force that could be transferred to, for example, a balloon or balloon catheter extending through loading tool 10. Thus, distal portion 12 may help reduce additional "contact" between the balloon and other portions of the system.

At least a portion of loading tool 10 may be configured to shift between a first or "elongated" configuration and a second or "shortened" configuration. For example, proximal portion 14 may be configured to shorten. This may include forming proximal portion 14 from a material that can be rolled upon itself, folded, crumpled, or otherwise shortened. This may also include forming proximal portion 14 from a relative thin sleeve of material that can be manipulated by a clinician so that loading tool 10 may be shortened.

In use, loading tool 10 may be disposed over a catheter shaft, for example the shaft of a balloon catheter. This may include disposing loading tool 10 over the balloon or otherwise disposing or advancing loading tool 10 over the balloon. When so positioned, loading tool 10 may be used to pass the balloon through a valve/introducer/dilator and, ultimately, into a body lumen. After the balloon catheter is disposed in the desired location, loading tool 10 may be proximally retracted from the valve/introducer/dilator. In addition, loading tool 10 may be shifted to the shortened configuration. This may include "shortening" proximal portion 14. These are just examples. Some additional details regarding this process and similar processes using similar and other loading tools are disclosed herein.

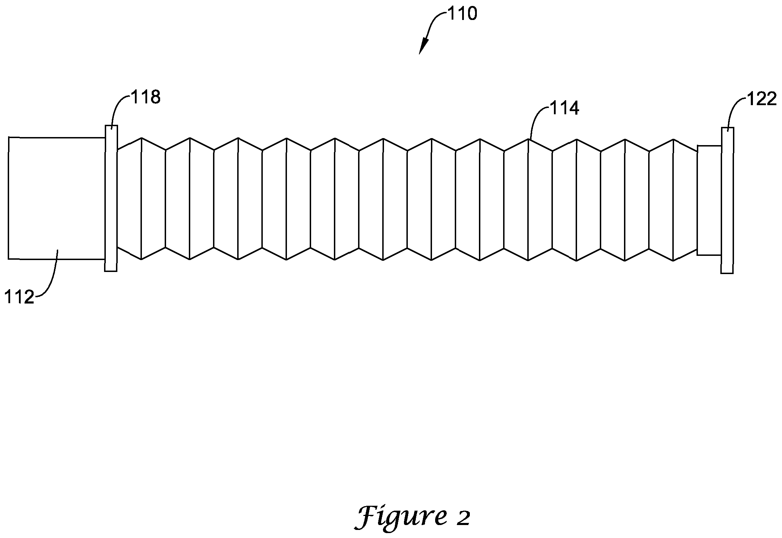

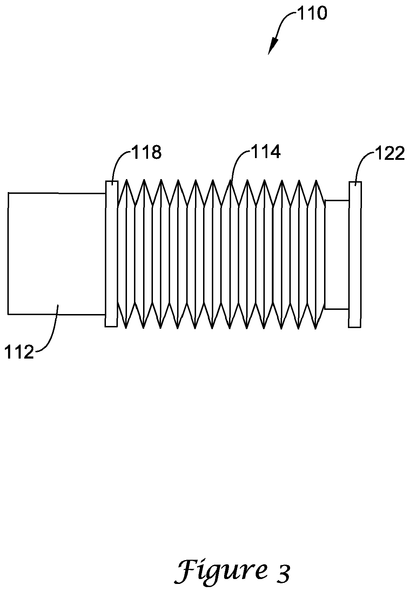

Figure 2 is a side view of another example loading tool 110 that may be similar in form and function to other loading tools disclosed herein. Loading tool 110 may include distal portion 112 having flange 118 and proximal portion 114 having flange 122. Just like loading tool 10, a portion of loading tool 110 may be configured to shift between a first or "elongated" configuration and a second or "shortened" configuration. For example, proximal portion 114 may include one or more collapsible corrugations or baffles formed therein. These corrugations may allow loading tool 110 to shift to a shortened configuration as shown in Figure 3. The extent to which loading tool 110 may be shortened may vary. For example, loading tool 110 may shorten to a length that is about 50% or less of the "original" (e.g., "unshortened") length, or about 40% or less of the original length, or about 30% or less of the original length, or about 20% or less of the original length, or about 10% or less of the original length. In one example, loading tool 110 may have an original, unshortened length that is can cover at least the full length of a balloon on a balloon catheter (e.g., a balloon having a length of about 200 mm or less, 150 mm or less, 100 mm or less, or another suitable length) and loading tool 110 may shorten to a length that is about 5 cm or less, or about 4 cm or less, or about 3 cm or less, or about 2 cm or less, or about 1 cm or less, or about 1 cm. These are just examples.

It should be noted that the corrugations shown in Figures 2-3 may have a variety of different forms. This may include changes to the number of corrugations, shape of the corrugations, the amount of the length of loading tool 110 that includes corrugations, etc. Indeed, it is contemplated that in at least some embodiments, the corrugations may be flattened out by elongating proximal portion 114. In doing so, proximal portion 114 may have a form similar to proximal portion 14. In addition, loading tool 10 may also include corrugations (e.g., along proximal portion 14) but the corrugations may not be visible due some elongation of proximal portion 14.

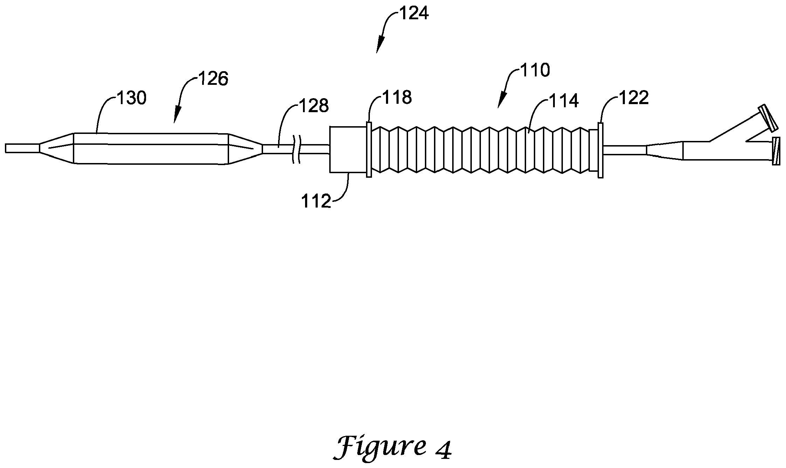

Figure 4 illustrates a medical device assembly 124 and, in general, portions of some of the methods for using loading tool 110 (and/or other loading tools disclosed herein). In this example, assembly 124 may include a balloon catheter or other suitable medical device 126. Balloon catheter 126 may include a catheter shaft 128 and a balloon 130 attached to catheter shaft 128. In at least some embodiments, balloon 130 may include a pharmaceutical coating. In some of these and in other embodiments, balloon 130 may include a stent or endoprosthesis disposed thereon. The stent or endoprosthesis may include a pharmaceutical coating. Balloon 130 may be configured to shift between a generally collapsed state (where balloon 130 may be in a folded configuration and have one or more wings or folds formed therein) and an expanded configuration. In Figure 4, balloon 130 is shown schematically in a collapsed or non-inflated state.

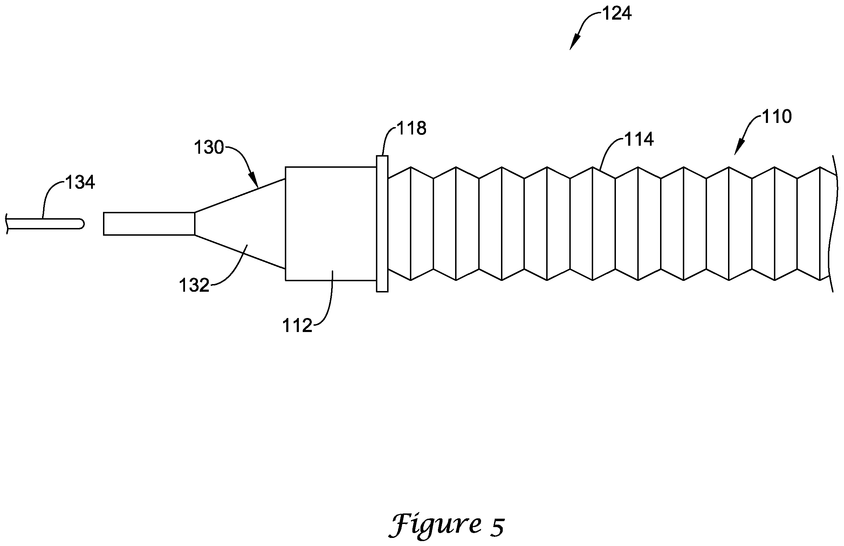

Loading tool 110 may be disposed about catheter shaft 128. This may include positioning loading tool 110 proximally of the balloon. Alternatively, loading tool 110 may be disposed about a portion of balloon 130. If loading tool 110 is not positioned about the balloon, loading tool 110 may be slid along catheter shaft 128 prior or during use so that at least a portion of loading tool 110 is disposed about balloon 130 as shown in Figure 5. This may include positioning loading tool 110 so that only a distal section or cone 132 of balloon 130 protrudes from distal portion 112. Such a configuration may allow a clinician to grasp the covered balloon 130 and load (e.g., "backload") a guidewire 134 into, for example, a guidewire lumen (not shown) of balloon catheter 126.

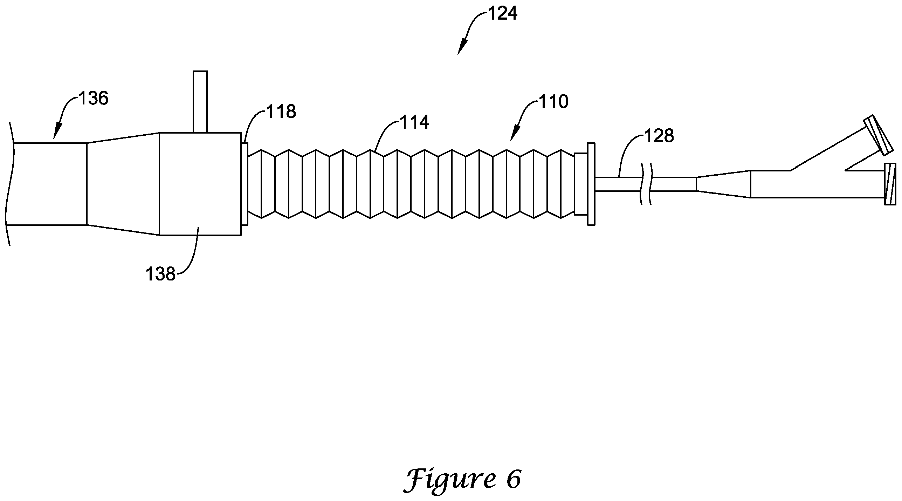

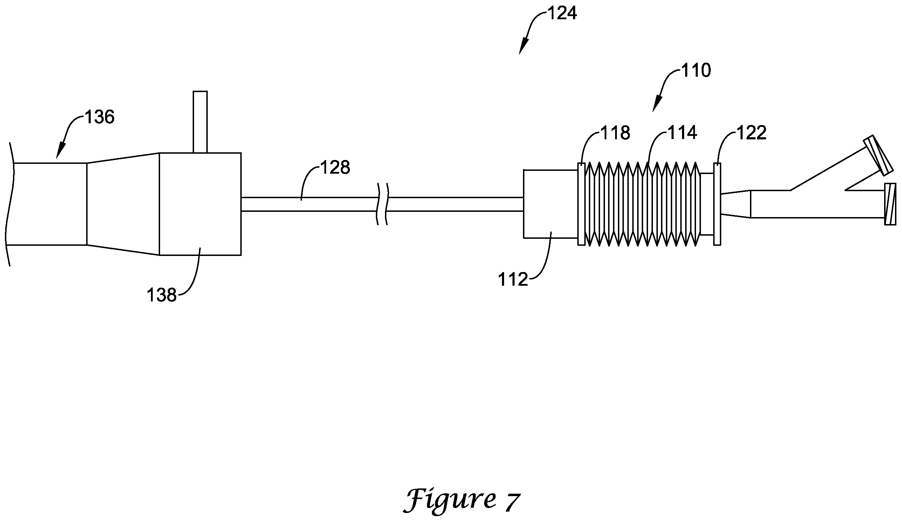

When suitably configured, balloon catheter 126 and loading tool 110 may be loaded into or otherwise advanced into a suitable introducer, dilator, or the like (e.g., introducer 136) as shown in Figure 6. This may include passing balloon catheter 126 and loading tool 110 through a valve 138 (e.g., a hemostasis valve, a touhy-borst valve, or the like). In doing so, flange 118 may abut valve 138, which may prevent loading tool 110 from passing further into valve 138 than desired. After at least a portion of balloon catheter 126 is positioned past valve 138 (e.g., after balloon 130 is advanced past valve 138), balloon 130 may be advanced relative to loading tool 110 into the entry site of a body lumen such as an introducer sheath for use. When balloon catheter 126 is positioned as desired, loading tool 110 may be proximally retracted from valve 138 and, if desired, shifted into a shortened configuration as shown in Figure 7. This may provide the clinician access to catheter shaft 128 so that balloon catheter 126 can be used for the desired intervention.

In some embodiments, loading tool 110 (and/or loading tool 10) may be removed from balloon catheter 126. This may include cutting, tearing, scoring, or otherwise splitting loading tool 110 so that it can be removed from balloon catheter 126. For example, in some embodiments, portions or all of loading tool 10/110 may include oriented polytetrafluoroethylene (e.g., longitudinally or axially oriented polytetrafluoroethylene). This may allow loading tool 10/110 to be split or otherwise torn longitudinally in order to remove loading tool 10/110 from another device such as a catheter shaft (e.g., catheter shaft 128).

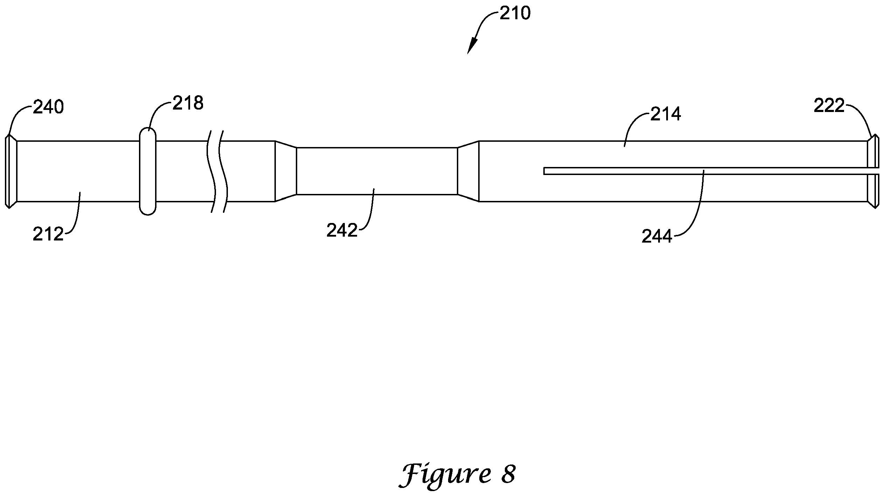

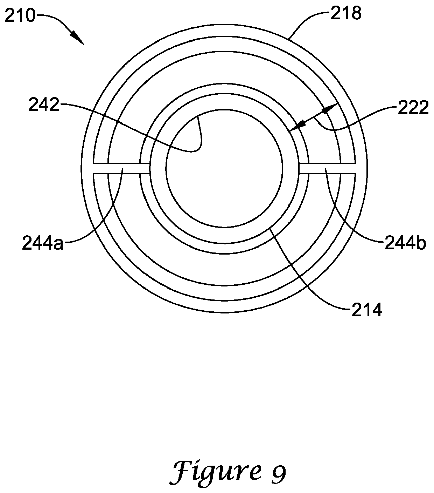

Figure 8 illustrates another example loading tool 210 that may be similar in form and function to other loading tools disclosed herein. Loading tool 210 may include distal portion 212 and proximal portion 214. In at least some embodiments, a necked down portion 242 may be disposed between distal portion 212 and proximal portion 214. Distal portion 212 may include a flared distal end 240 and a flange 218. Flared end 240 may aid in guiding a balloon or other device into distal portion 212 when loading tool 210 is slid over the balloon. Flange 218 may serve to prevent loading tool 210 from inadvertently passing into a valve or introducer. Proximal portion 214 may include a flared proximal end 222. One or more slots 244 may be formed in loading tool 210. For example, proximal portion 214 may have a pair of slots 244a/244b as shown in Figure 9.

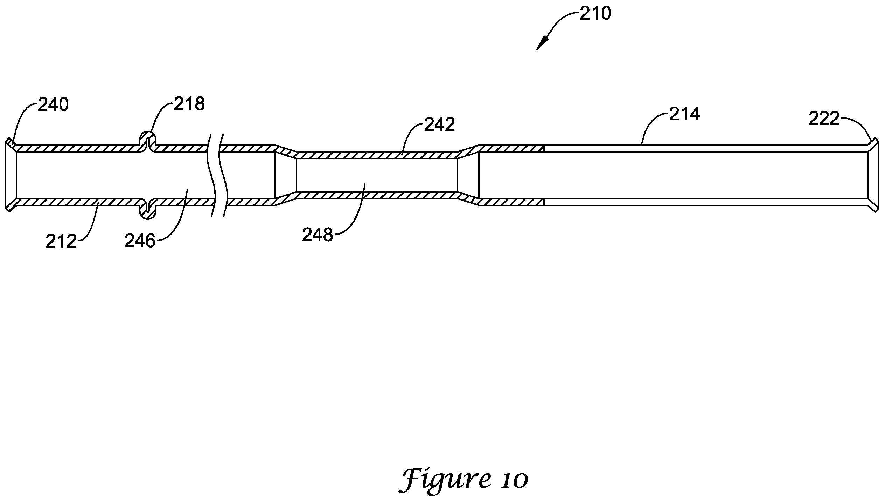

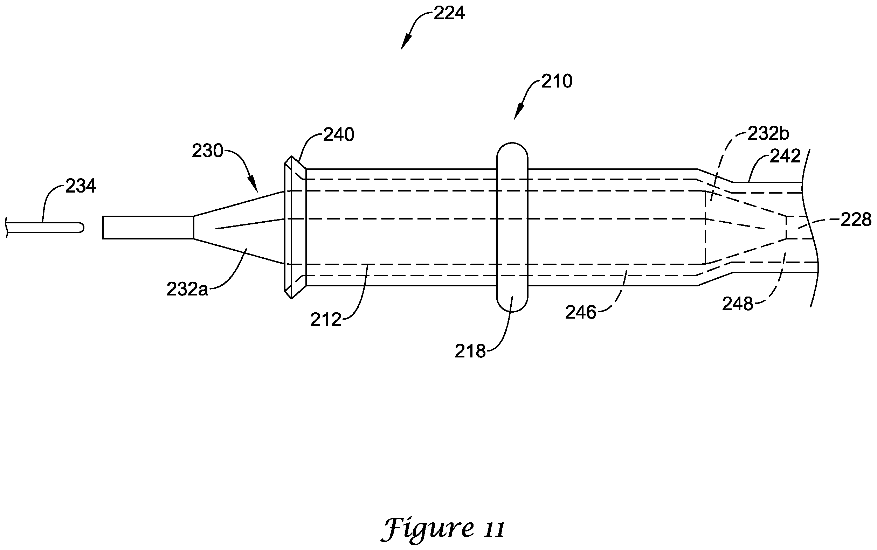

Distal portion 212 may define a lumen 246 as shown in Figure 10. Similarly, necked down portion 242 may define a lumen 248. In general, lumen 248 may be generally narrower than lumen 246. This may allow balloon 230 to be contained within lumen 246 while lumen 248 may prevent balloon 230 from moving proximally of necked down portion 242. For example, Figure 11 illustrates assembly 224 where distal portion 212 is disposed over balloon 230. Balloon 230, which is shown schematically, may be in a collapsed or folded configuration within loading tool 210. Distal cone portion 232a of balloon 230 may protrude from distal portion 212 of loading tool 210. Necked down portion 242 and/or lumen 248 may define a stop that prevents proximal migration of balloon 230. For example, a proximal cone portion 232b may catch at necked down portion 242. Catheter shaft 228 may pass through necked down portion 242 and through proximal portion 214.

When arranged as shown in Figure 11, guidewire 234 may be backloaded and assembly 224 may be advanced through a medical valve in a manner similar to what is described above in relation to Figure 6. For example, loading tool 210 may be passed through a valve (e.g., a hemostasis valve, a touhy-borst valve, or the like). In doing so, flange 218 may abut the valve and prevent loading tool 210 from passing further into the valve than desired. Balloon 230 may be advanced through loading tool 210 and into the entry site of a body lumen such as an introducer sheath for use. Loading tool 210 may also be proximally retracted from the valve.

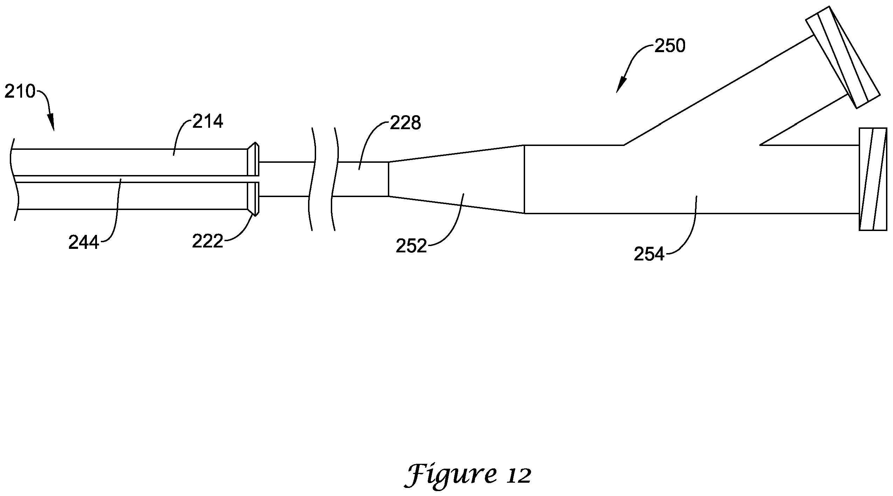

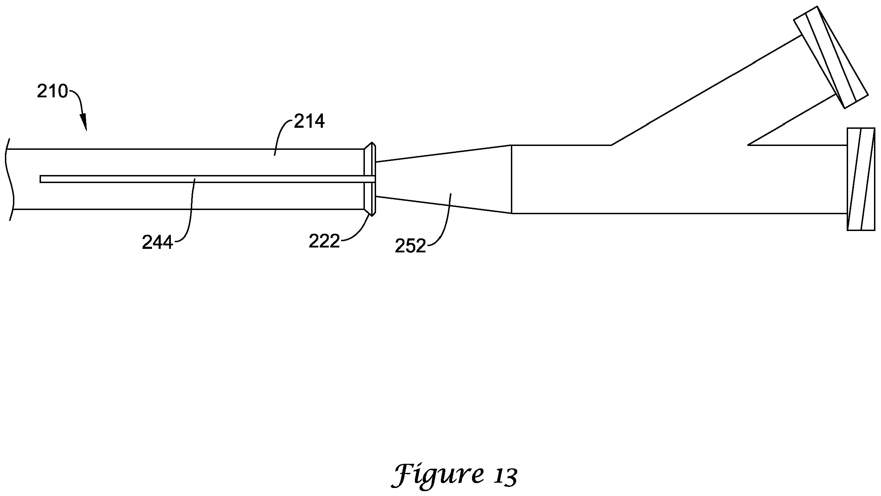

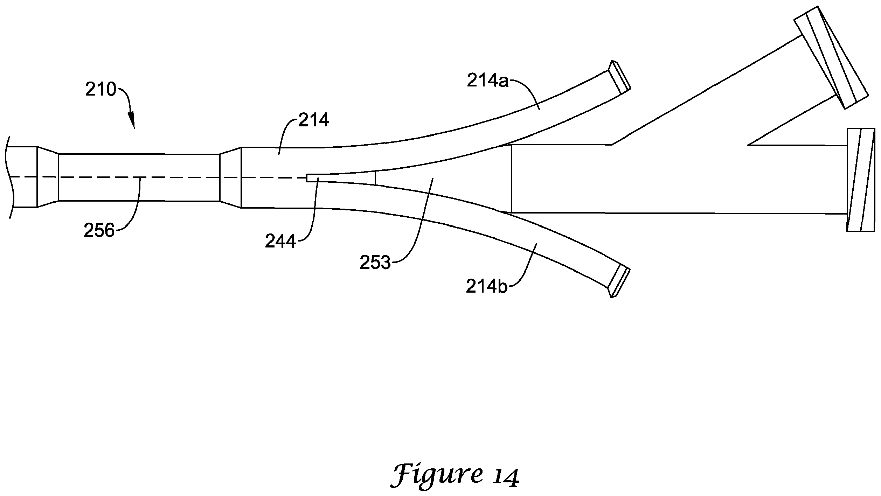

In at least some embodiments, slots 244 may be used to peel away or otherwise split loading tool 210 in a manner that may permit loading tool 210 to be removed from catheter shaft 228. For example, Figure 12 illustrates a hub assembly 250 attached to catheter shaft 228. Hub assembly 250 may include a strain relief 252 and a y-adapter 254. Loading tool 210 may be proximally retracted to a position adjacent to strain relief 252 as shown in Figure 13. Further proximal retraction of loading tool 210 may cause slots 244 to open and define an enlarged opening 253 as shown in Figure 14. In doing so, proximal portion 214 of loading tool 210 to split into segments 214a/214b. To facilitate splitting, portions or all of loading tool 210 may include longitudinally-oriented polytetrafluoroethylene. This may allow loading tool 210 to more easily tear along the longitudinal axis. In some of these and in other embodiments, loading tool 210 may include a line 256 that takes the form of a scored line (e.g., which may be scored with a laser, mechanically, or using other suitable devices), a line of weakness (e.g., which may correspond to about 40-80% of the axial thickness of the loading tool 210 removed), perforations, or the like. Line 256 may correspond to a portion of loading tool 210 that is designed to tear more easily. Line 256 may extend along a portion of the length of loading tool 210 or along the full length of loading tool 210.

While not intending to be limiting, a variety of different dimensions are contemplated for loading tool 210. Some of the dimensions that are contemplated are disclosed herein. Distal portion 212 may have a length of about 50-300 mm, or about 100-200 mm, or about 125-175 mm, or about 155 mm. In general, distal portion 212 may have a length suitable to contain a balloon. For example, distal portion 212 may have a length of about 200 mm when used with a 200 mm balloon, or about 155 mm when used with a 155 mm balloon, and so on. Flange 218 may be positioned about 20 mm or less from the distal end of distal portion 212, or about 15 mm or less from the distal end of distal portion 212, or about 10 mm or less from the distal end of distal portion 212, or about 10 mm from the distal end of distal portion 212. Proximal portion 214 may have a length of about 5-30 mm, or about 10-20 mm, or about 15 mm. Flared ends 240/222 may flare out at an angle of about 15-75 degrees, or about 20-60 degrees, or about 30-50 degrees, or about 40 degrees. The outer diameter of flange 218 may be about 1.27 mm to 7.62 mm (0.05 to 0.30 inches), or about 0.254 mm to 0.508 mm (0.1 to 0.2 inches), or about 3.3 mm (0.130 inches) or less. Necked down portion 242 may have a length of about 1-10 mm, or about 2-7 mm, or about 3-5 mm. These dimensions are just examples and may vary when loading tool 210 is used with differently sized balloons.

The materials that can be used for the various components of loading tool 10 (and/or other loading tools disclosed herein) and the various tubular members disclosed herein may include those commonly associated with medical devices. For simplicity purposes, the following discussion makes reference to loading tool 10. However, this is not intended to limit the devices and methods described herein, as the discussion may be applied to other similar tubular members and/or components of tubular members or devices disclosed herein.

Loading tool 10 may be made from a metal, metal alloy, polymer (some examples of which are disclosed below), a metal-polymer composite, ceramics, combinations thereof, and the like, or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN® available from DuPont), polyether block ester, polyurethane (for example, Polyurethane 85A), polypropylene (PP), polyvinylchloride (PVC), polyether-ester (for example, ARNITEL® available from DSM Engineering Plastics), ether or ester based copolymers (for example, butylene/poly(alkylene ether) phthalate and/or other polyester elastomers such as HYTREL® available from DuPont), polyamide (for example, DURETHAN® available from Bayer or CRISTAMID® available from Elf Atochem), elastomeric polyamides, block polyamide/ethers, polyether block amide (PEBA, for example available under the trade name PEBAX®), ethylene vinyl acetate copolymers (EVA), silicones, polyethylene (PE), Marlex high-density polyethylene, Marlex low-density polyethylene, linear low density polyethylene (for example REXELL®), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR®), polysulfone, nylon, nylon-12 (such as GRILAMID® available from EMS American Grilon), perfluoro(propyl vinyl ether) (PFA), ethylene vinyl alcohol, polyolefin, polystyrene, epoxy, polyvinylidene chloride (PVdC), poly(styrene-*b*-isobutylene-*b-*styrene) (for example, SIBS and/or SIBS 50A), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

In at least some embodiments, portions or all of loading tool 10 may also be doped with, made of, or otherwise include a radiopaque material. Radiopaque materials are understood to be materials capable of producing a relatively bright image on a fluoroscopy screen or another imaging technique during a medical procedure. This relatively bright image aids the user of loading tool 10 in determining its location. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of loading tool 10 to achieve the same result.

In some embodiments, a degree of Magnetic Resonance Imaging (MRI) compatibility is imparted into loading tool 10. For example, loading tool 10, or portions thereof, may be made of a material that does not substantially distort the image and create substantial artifacts (i.e., gaps in the image). Certain ferromagnetic materials, for example, may not be suitable because they may create artifacts in an MRI image. Loading tool 10, or portions thereof, may also be made from a material that the MRI machine can image. Some materials that exhibit these characteristics include, for example, tungsten, cobalt-chromium-molybdenum alloys (e.g., UNS: R30003 such as ELGILOY®, PHYNOX®, and the like), nickel-cobalt-chromium-molybdenum alloys (e.g., UNS: R30035 such as MP35-N® and the like), nitinol, and the like, and others.

It should be understood that this disclosure is, in many respects, only illustrative. Changes may be made in details, particularly in matters of shape, size, and arrangement of steps without exceeding the scope of the disclosure.

## Claims

1. A loading tool (10, 110, 210) for use with a medical device, the loading tool (10, 110, 210) comprising:
a tubular member configured to be disposed about a balloon catheter (126), the tubular member including a distal portion (12, 112, 212), a proximal portion (14, 114, 214), and a necked down region (242) disposed between the distal portion (12, 112, 212) and the proximal portion (14, 114, 214);
wherein the distal portion (12, 112, 212) has a distal inner diameter and the necked down region (242) includes a necked inner diameter smaller than the distal inner diameter; and
**characterized in that** the proximal portion (14, 114, 214) has a plurality of axial slits formed therein, wherein the axial slits are in the form of slots (244, 244a, 244b).

2. The loading tool of claim 1, wherein the tubular member includes a longitudinally-oriented material.

3. The loading tool of claim 2, wherein the tubular member includes oriented polytetrafluoroethylene.

4. The loading tool of claim 1, wherein the plurality of axial slits includes a pair of opposed slits formed at a proximal end of the proximal portion (14, 114, 214).

5. The loading tool of claim 1, wherein the distal portion (12, 112, 212) includes a flared distal end (240).

6. The loading tool of claim 1, wherein the distal portion (12, 112, 212) includes a distal flange (218).

7. The loading tool of claim 1, wherein the tubular member has a scored line formed therein.

8. The loading tool of claim 7, wherein the scored line includes a mechanically scored line.

9. The loading tool of claim 7, wherein the scored line includes a laser scored line.

10. A medical device assembly, comprising:
a balloon catheter (126) including a catheter shaft and a balloon (130) coupled to the catheter shaft (128); and
a loading tool (10, 110, 210) slidably disposed on the catheter shaft (128), the loading tool (10, 110, 210) including:
a tubular member including a distal portion (12, 112, 212), a proximal portion (14, 114, 214), and a necked down region (242) disposed between the distal portion (12, 112, 212) and the proximal portion (14, 114, 214),
wherein the distal portion (12, 112, 212) has a distal inner diameter and the necked down region (242) includes a necked inner diameter smaller than the distal inner diameter, and
**characterized in** the proximal portion (14, 114, 214) has a plurality of axial slits formed therein,
wherein the axial slits are in the form of slots (244, 244a, 244b).

11. The assembly of claim 10, wherein the tubular member includes a longitudinally-oriented material.

12. The assembly of claim 10, wherein the tubular member includes oriented polytetrafluoroethylene.

13. The assembly of claim 10, wherein the plurality of axial slits includes a pair of opposed slits formed at a proximal end of the proximal portion (14, 114, 214).

14. The assembly of claim 10, wherein the distal portion (12, 112, 212) includes a flared distal end (240).

15. The assembly of claim 10, wherein the distal portion (12, 112, 212) includes a distal flange (218).

## Patentansprüche

1. Ladewerkzeug (10, 110, 210) zur Verwendung mit einer medizinischen Vorrichtung, wobei das Ladewerkzeug (10, 110, 210) das Folgende umfasst:
ein röhrenförmiges Element, das zur Anordnung um einen Ballonkatheter (126) ausgelegt ist, wobei das röhrenförmige Element einen distalen Abschnitt (12, 112, 212), einen proximalen Abschnitt (14, 114, 214) und einen verengten Bereich (242), der zwischen dem distalen Abschnitt (12, 112, 212) und dem proximalen Abschnitt (14, 114, 214) angeordnet ist, aufweist;
wobei der distale Abschnitt (12, 112, 212) einen distalen Innendurchmesser aufweist und der verengte Bereich (242) einen verengten Innendurchmesser aufweist, der kleiner als der distale Innendurchmesser ist; und
**dadurch gekennzeichnet, dass** der proximale Abschnitt (14, 114, 214) eine Vielzahl von darin ausgebildeten axialen Schlitzen aufweist, wobei die axialen Schlitze die Form von Längsschlitzen (244, 244a, 244b) aufweisen.

2. Ladewerkzeug nach Anspruch 1, wobei das röhrenförmige Element ein längs orientiertes Material aufweist.

3. Ladewerkzeug nach Anspruch 2, wobei das röhrenförmige Element orientiertes Polytetrafluorethylen aufweist.

4. Ladewerkzeug nach Anspruch 1, wobei die Vielzahl von axialen Schlitzen zwei gegenüberliegender Schlitze aufweist, die an einem proximalen Ende des proximalen Abschnitts (14, 114,214) ausgebildet sind.

5. Ladewerkzeug nach Anspruch 1, wobei der distale Abschnitt (12, 112, 212) ein aufgeweitetes distales Ende (240) aufweist.

6. Ladewerkzeug nach Anspruch 1, wobei der distale Abschnitt (12, 112, 212) einen distalen Flansch (218) aufweist.

7. Ladewerkzeug nach Anspruch 1, wobei das röhrenförmige Element eine darin ausgebildete Kerblinie aufweist.

8. Ladewerkzeug nach Anspruch 7, wobei die Kerblinie eine mechanisch eingeritzte Linie aufweist.

9. Ladewerkzeug nach Anspruch 7, wobei die Kerblinie eine mittels Laser eingeritzte Linie aufweist.

10. Medizinische Vorrichtungsanordnung, umfassend:
einen Ballonkatheter (126) mit einem Katheterschaft und einem mit dem Katheterschaft (128) gekoppelten Ballon (130); und
ein Ladewerkzeug (10, 110, 210), das verschiebbar auf dem Katheterschaft (128) angeordnet ist, wobei das Ladewerkzeug (10, 110, 210) das Folgende aufweist:
ein röhrenförmiges Element, das einen distalen Abschnitt (12, 112, 212), einen proximalen Abschnitt (14, 114, 214) und einen verengten Abschnitt (242), der zwischen dem distalen Abschnitt (12, 112, 212) und dem proximalen Abschnitt (14, 114, 214) angeordnet ist, aufweist,
wobei der distale Abschnitt (12, 112, 212) einen distalen Innendurchmesser aufweist und der verengte Bereich (242) einen verengten Innendurchmesser aufweist, der kleiner als der distale Innendurchmesser ist, und
**dadurch gekennzeichnet, dass** der proximale Abschnitt (14, 114, 214) eine Vielzahl von darin ausgebildeten axialen Schlitzen aufweist,
wobei die axialen Schlitze die Form von Längsschlitzen (244, 244a, 244b) aufweisen.

11. Anordnung nach Anspruch 10, wobei das röhrenförmige Element ein längs orientiertes Material aufweist.

12. Anordnung nach Anspruch 10, wobei das röhrenförmige Element orientiertes Polytetrafluorethylen aufweist.

13. Anordnung nach Anspruch 10, wobei die Vielzahl von axialen Schlitzen zwei gegenüberliegender Schlitze aufweist, die an einem proximalen Ende des proximalen Abschnitts (14, 114, 214) ausgebildet sind.

14. Anordnung nach Anspruch 10, wobei der distale Abschnitt (12, 112, 212) ein aufgeweitetes distales Ende (240) aufweist.

15. Anordnung nach Anspruch 10, wobei der distale Abschnitt (12, 112, 212) einen distalen Flansch (218) aufweist.

## Revendications

1. Outil de chargement (10, 110, 210) destiné à être utilisé avec un dispositif médical, l'outil de chargement (10, 110, 210) comprenant :
un élément tubulaire configuré pour être disposé autour d'un cathéter à ballonnet (126), l'élément tubulaire comprenant une partie distale (12, 112, 212), une partie proximale (14, 114, 214), et une région de col (242) disposée entre la partie distale (12, 112, 212) et la partie proximale (14, 114, 214) ;
la partie distale (12, 112, 212) ayant un diamètre intérieur distal et la région de col (242) comprenant un diamètre intérieur de col plus petit que le diamètre intérieur distal ; et
**caractérisé en ce que** la partie proximale (14, 114, 214) a une pluralité de fentes axiales formées dans celle-ci, les fentes axiales étant sous la forme de fentes (244, 244a, 244b).

2. Outil de chargement selon la revendication 1, l'élément tubulaire comprenant un matériau orienté longitudinalement.

3. Outil de chargement selon la revendication 2, l'élément tubulaire comprenant du polytétrafluoroéthylène orienté.

4. Outil de chargement selon la revendication 1, la pluralité de fentes axiales comprenant une paire de fentes opposées formées au niveau d'une extrémité proximale de la partie proximale (14, 114, 214).

5. Outil de chargement selon la revendication 1, la partie distale (12, 112, 212) comprenant une extrémité distale évasée (240).

6. Outil de chargement selon la revendication 1, la partie distale (12, 112, 212) comprenant une bride distale (218).

7. Outil de chargement selon la revendication 1, l'élément tubulaire ayant une ligne rainurée formée dans celui-ci.

8. Outil de chargement selon la revendication 7, la ligne rainurée comprenant une ligne rainurée mécaniquement.

9. Outil de chargement selon la revendication 7, la ligne rainurée comprenant une ligne rainurée au laser.

10. Ensemble de dispositif médical, comprenant :
un cathéter à ballonnet (126) comprenant une tige de cathéter et un ballonnet (130) couplé à la tige de cathéter (128) ; et
un outil de chargement (10, 110, 210) disposé de manière coulissante sur la tige de cathéter (128), l'outil de chargement (10, 110, 210) comprenant :
un élément tubulaire comprenant une partie distale (12, 112, 212), une partie proximale (14, 114, 214) et une région de col (242) disposée entre la partie distale (12, 112, 212) et la partie proximale (14, 114, 214),
la partie distale (12, 112, 212) ayant un diamètre intérieur distal et la région de col (242) comprenant un diamètre intérieur de col plus petit que le diamètre intérieur distal, et
**caractérisé en ce que** la partie proximale (14, 114, 214) a une pluralité de fentes axiales formées dans celle-ci,
les fentes axiales étant sous la forme de fentes (244, 244a, 244b).

11. Ensemble selon la revendication 10, l'élément tubulaire comprenant un matériau orienté longitudinalement.

12. Ensemble selon la revendication 10, l'élément tubulaire comprenant du polytétrafluoroéthylène orienté.

13. Ensemble selon la revendication 10, la pluralité de fentes axiales comprenant une paire de fentes opposées formées au niveau d'une extrémité proximale de la partie proximale (14, 114, 214).

14. Ensemble selon la revendication 10, la partie distale (12, 112, 212) comprenant une extrémité distale évasée (240).

15. Ensemble selon la revendication 10, la partie distale (12, 112, 212) comprenant une bride distale (218).
